(19) Europäisches Patentamt
European Patent Office
Office européen des brevets

(11) EP 3 188 720 B1

# (12) EUROPEAN PATENT SPECIFICATION

(45) Date of publication and mention of the grant of the patent:
**21.04.2021 Bulletin 2021/16**

(21) Application number: **15838561.7**

(22) Date of filing: **31.08.2015**

(51) Int Cl.:
***A61K 9/00*** (2006.01) ***A61K 9/70*** (2006.01)
***A61K 31/4985*** (2006.01) ***A61P 15/10*** (2006.01)

(86) International application number:
**PCT/KR2015/009151**

(87) International publication number:
**WO 2016/036093 (10.03.2016 Gazette 2016/10)**

## (54) TADALAFIL ORAL DISPERSIBLE FILM AND PREPARING METHOD THEREOF

ORALER DISPERGIERBARER TADALAFILFILM UND HERSTELLUNGSVERFAHREN DAFÜR

FILM DISPERSIBLE ORAL DE TADALAFIL ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2014 KR 20140115985**

(43) Date of publication of application:
**12.07.2017 Bulletin 2017/28**

(73) Proprietor: **Seoul Pharma. Co., Ltd.**
**Siheung-si, Gyeonggi-do 429-848 (KR)**

(72) Inventors:
- **CHO, Yong Hee**
**Bucheon-si**
**Gyeonggi-do 14586 (KR)**
- **JANG, Won Young**
**Siheung-si**
**Gyeonggi-do 14693 (KR)**
- **UM, Jin Hee**
**Ansan-si**
**Gyeonggi-do 15486 (KR)**
- **CHOE, Dong Woon**
**Suwon-si**
**Gyeonggi-do 16336 (KR)**
- **KIM, Hyun Soo**
**Siheung-si**
**Gyeonggi-do 15002 (KR)**
- **KIM, Jung Hoon**
**Seongnam-si**
**Gyeonggi-do 13599 (KR)**

(74) Representative: **D Young & Co LLP**
**120 Holborn**
**London EC1N 2DY (GB)**

(56) References cited:
**EP-A1- 3 111 929**
**WO-A1-2014/091134**
**WO-A2-2012/053006**
**KR-A- 20080 023 873**
**KR-A- 20100 012 867**
**KR-A- 20100 052 453**
**US-A1- 2010 099 687**
**US-A1- 2010 240 724**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 188 720 B1

## Description

[Technical Field]

The present disclosure relates to a composition for preparing an oral dispersible film comprising a tadalafil or its pharmaceutically acceptable salt thereof, a surfactant, a plasticizer and a sweetening agent; an oral dispersible film; and a preparing method thereof.

[Background Art]

There are various types of orally dispersible preparations such as tablets, chewable tablets, sublingual tablets, capsules and liquid formulations for oral administration. Within those preparations, general tablets and capsules among others are disadvantageous for patients having difficulties in taking drugs and liquid formulations are disadvantageous in light of stability and inaccurate volume amount. Therefore, necessity of a new and easily administrable preparation is arising.

Recently, some formulations having different drug delivery systems are released. ODT (oral dispersible tablet) which is an oral dispersible solid preparation is one of those formulations. However, the problems are that ODT generally does not completely and consistently disintegrated in a short amount of time and frequently requires water after dose.

The widely mentioned oral dispersible film is a newly proposed preparation for resolution of the above problems. This oral dispersible film preparation has some advantages that previous solid, liquid formulations and ODT do not have. The oral dispersible film preparation is useful for the elderly having a trouble with taking tablets or capsules as well as children, physically challenged person, patients on a bed and busy people because it is administrable without water, and it is assessed as the most improved type of drug disintegration compared to any preceding formulations.

Especially, it is advantageous that the oral dispersible film is applicable to hepatic metabolism of drugs among drugs absorbed from alimentary canal because hepatic first pass can be evaded when drug is absorbed into oral mucosa. Therefore, various attempts are being made to produce oral dispersible film preparation for improving property of the film and drug compliance of patients.

Although the oral dispersible film has the virtue, the drug has low initial dissolution and dissolution rate compared to the general tablet or the ODT, and these demerits are blocking development of generic drugs having bioequivalence and incrementally modified drugs.

Specifically, macromolecules such as hydroxypropyl methylcellulose, pullulan, polyvinyl acetate, polyethylene oxide, gelatin and alginic acid among others that are used as a film forming agent due to characteristics of the oral dispersible film are used a lot as a film formulation of the oral dispersible film in the amount of 10-90 wt% based on total solid content by exhibiting its fine capability in film formation, but these macromolecules blocks development of formulations because of its characteristics as decrement of disintegration rate and dissolution rate according to increment of contents and severer decrement of degree of dissolution rate in case when pharmaceutical active ingredients are poorly water-soluble.

In addition, preventing admixture of harmful to the human body or unnecessary substances other than main (active) ingredient in formulations is important for quality control on pharmaceuticals. The impurities expected to be admixed to pharmaceuticals are inorganic impurities, residual solvents, water and related compounds among others and the related compounds mean raw materials for synthesizing pharmaceuticals, intermediate products and resolvents produced during storage, and these are known as complicate to separate from main ingredient because of its structural similarity. Therefore, inhibiting generation of the related compounds during distribution or storage as well as preparation step of pharmaceuticals is an important factor in evaluating pharmaceutical development.

The tadalafil, an active ingredient of Cialis®, is known to have therapeutic effects of male impotence. Chemical name of the tadalafil is (6R-trans)-6-(1,3-Benzodioxol-5-yl)-2,3,6,7,12,12a-hexahydro-2-methyl-pyrazino[1',2':1,6]pyrido[3,4-b]indole-1,4-dione. Chemical structure of the tadalafil (CAS# 171596-29-5) is illustrated below:

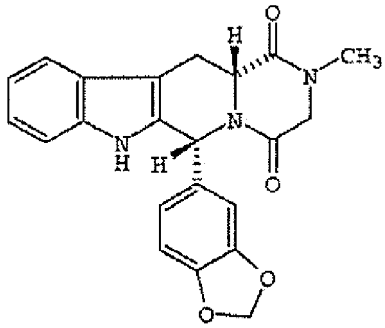

Tadalafil is a solid known as substantially water insoluble and poorly soluble to some organic solvents such as methanol, ethanol and acetone among others. U.S. Patent No. 6,841,167 teaches that it has water solubility of about 2 $\mu$g per 1 mL of water at 25°C.

Therefore controlling dissolution rate of drug is very important for effective absorption of the tadalafil which has low water solubility but technical difficulties lie in application of the tadalafil to the oral dispersible film preparation because the oral dispersible film preparation has low dissolution rate.

KR 2008 0023873 describes oral consumable films comprising a film-forming agent, trehalose and tadalafil, a flavoring agent, a filler, a softener, a stabilizer, a saliva-stimulating agent and a sweetener.

WO 2012/053006 describes an oral film having reduced adherence to oral cavity comprising a water-soluble polymer in combination with film former that is suitable for delivering drugs from various pharmacological categories ranging from conventional small molecules to proteins or peptides, wherein the ratio of water-soluble polymer to the film former is about 25:1 to about 250:1. This document describes that tadalafil may be delivered by the films disclosed therein.

US 2010/240724 describes an edible film composition comprising a waxy starch hydrolysate, a modified starch and a water-soluble polymer. This document discloses that the edible film may comprise tadalafil.

WO 2014/091134 describes an oral and/or buccal composition in the form of a thin film of a pharmaceutically active ingredient weakly soluble in water and gastro-intestinal tract fluids. Tadalafil is described as a suitable drug for use in the films described therein.

EP 3111929 is a patent application that was published after the priority date of the present application, and which discloses a tadalafil-containing orally disintegrating film formulation comprising a combination of pullulan and polyvinylpyrrolidone as a film forming agent and a process for preparing the same.

As explained above, there is a necessity of a formulation which effectively improves dissolution rate of a drug and inhibits related compounds at the same time in light of producing a recently emerging oral dispersible film, especially an oral dispersible film including a poorly water-soluble tadalafil as an active ingredient but no such improved tadalafil oral dispersible film exists so far.

Additionally, mass production is required for commercial utilization but occasionally reduction of product value incurs due to film breakage, excessive flexibility and leakage of liquid additives for films to the surface of a film in the course of distribution. These breakage, flexibility and oil leakage among others are deemed to be the problems to be overcome for commercial use.

The above information disclosed in this Background section is only for enhancement of understanding of the background of the invention and it may therefore contain information that does not form the prior art that is already known to a person of ordinary skill in the art.

[Disclosure of Invention]

[Technical Problem]

The inventors of the present disclosure discovered that dissolution rate of the tadalafil in a film formulation could be markedly improved without complicated processes through proper adjustment of constituting ingredients and related compounds could be inhibited while researching tadalafil oral dispersible film preparation which can improve dissolution rate of a drug and minimize generation of related compounds, and accordingly completed the present disclosure. They further discovered that product value can be increased by maintaining storage stability of a film in the course of distribution, breakage prevention which is the basic property of mass production can be obtained, flexibility can be secured and oil leakage can be prevented via proper adjustment of constituting ingredients, and accordingly completed the present disclosure.

Therefore, an object of the present disclosure is to provide a composition for preparing an oral dispersible film comprising a tadalafil or its pharmaceutically acceptable salt thereof, a surfactant, a plasticizer and a sweetening agent,

an oral dispersible film and a preparing method thereof.

[Solution to Problem]

To achieve the above object, the present disclosure provides the composition for preparing an oral dispersible film comprising a tadalafil or its pharmaceutically acceptable salt thereof, a surfactant, a plasticizer and a sweetening agent, wherein the surfactant is sodium lauryl sulfate (SLS) and polysorbate, the plasticizer is glycerin and polyethylene glycol and the sweetening agent is sorbitol solution.

Furthermore, the present disclosure provides the oral dispersible film prepared by drying the above composition.

Furthermore, the present disclosure provides the method for preparing the oral dispersible film comprising (a) preparing an agitation solution by adding and agitating the tadalafil or its pharmaceutically acceptable salt thereof, the surfactant, a binding agent, the plasticizer, a disintegrant and the sweetening agent into a solvent; (b) preparing a film solution by homogenizing or comminuting the agitation solution with a homomixer, and agitating the same with an addition of the film forming agent; (c) molding the film by drying the film solution, wherein the surfactant is sodium lauryl sulfate (SLS) and polysorbate, the plasticizer is glycerin and polyethylene glycol and the sweetening agent is sorbitol solution.

[Advantageous Effects of Invention]

The oral dispersible film according to the present disclosure has superior stability compared to the previous cialis tablets because it can improve the dissolution rate of the tadalafil without complicated processes and generation of the related compounds are inhibited regardless of existence of a packaging. Also, superior film properties including prevention of film breakage, securement of flexibility and prevention of oil leakage from film formulation can be maintained when comprising specific content of a polyethylene glycol 400 as the plasticizer.

[Mode for the Invention]

The present disclosure provides a composition for preparing an oral dispersible film comprising a tadalafil or its pharmaceutically acceptable salt thereof, a surfactant, a plasticizer and a sweetening agent, wherein the surfactant is sodium lauryl sulfate (SLS) and polysorbate, the plasticizer is glycerin and polyethylene glycol and the sweetening agent is sorbitol solution.

The composition for preparing the oral dispersible film according to the present disclosure has superiority in production of the tadalafil oral dispersible film that dissolution rate of the tadalafil is improved and related compounds are inhibited regardless of existence of a packaging without complicated processes.

The composition for preparing the oral dispersible film according to the present disclosure has superior storage stability because it is designed to prevent tadalafil film breakage during distribution or decrease in flexibility and oil leakage.

The "tadalafil" is a phosphodiesterase (PDE) 5 inhibitor and the present disclosure includes not only the tadalafil but also solvates and hydrates thereof.

The "pharmaceutically acceptable salt" is salts prepared with an acid or base having none or less toxicity. A pharmaceutically acceptable base addition salt includes lithium, natrium, potassium, calcium, ammonium, magnesium and organic amine salt but not limited to these.

A pharmaceutically acceptable acid addition salt includes salts formed of propionic acid, isobutyl acid, oxalic acid, malic acid, malonic acid, benzoic acid, succinic acid, suberic acid, fumaric acid, manderic acid, phthalic acid, benzene sulfonic acid, p-tolyl sulfonic acid, citric acid, tartaric acid, methane sulfonic acid, hydrochloric acid, bromic acid, nitric acid, carbonic acid, monohydrogen carbonic acid, phosphoric acid, monohydrogen phosphoric acid, dihydrogen phosphoric acid, sulfuric acid, monohydrogen sulfuric acid, hydrogen iodide and phosphorous acid among others but not limited to these. Also comprising amino acid salts such as alginate and analogues of organic acid such as glucuronic acid and galactunoric acid but not limited to these.

The "oral dispersible film (ODF)" also called as strip or orally dissolving film and it is a film formulation that can be taken via dissolution or minute dispersion in an oral cavity. These films usually placed on the tongue to be dissolved but also can be administered by adhere films to palate, sublingual region or buccal cavity. The film formulation according to the present disclosure is advantageous in that it is administrable without water.

The surfactant is sodium lauryl sulfate (SLS) and polysorbate. Polysorbate 20 (monolaurate), 40 (monopalmitate), 60 (monostearate), 65 (tristearate) and 80 (monooleate) are usable as the polysorbate without restriction but the polysorbate 20 is most preferable.

In addition, plasticizers which do not affect physical properties of the film can be used. The plasticizer used in the composition of the present invention is glycerin and polyethylene glycol.

In addition, the sweetening agent is sorbitol solution.

In addition, it is preferable that the surfactant can be sodium lauryl sulfate (SLS) and polysorbate 20, the

plasticizer can be glycerin and polyethylene glycol, and the sweetening agent can be sorbitol solution for the composition for preparing the oral dispersible film.

The surfactant can be included as 1-30 wt%, preferably 1-20 wt%, and more preferably 1-10 wt% based on the total weight of solid contents of the film.

Also, the plasticizer can be included as 1-25 wt%, preferably 1-20 wt%, and more preferably 1-15 wt% based on total weight of solid contents of the film.

Especially when the plasticizer is polyethylene glycol 400, it is preferable to be included as 1-7 wt% based on total weight of solid contents of the film.

Additionally, the sweetening agent can be included as 1-50 wt%, preferably 1-40 wt%, and more preferably 1-30 wt% based on total weight of solid contents of the film.

The "total weight of solid contents of the film" is aggregate of weight of the solid contents comprising surfactant, tadalafil, binding agent, plasticizer, disintegrant, sweetening agent, fragrance ingredient and coloring agent among others which are compositions for preparing the tadalafil oral dispersible film wherein solvents are excluded.

Upon changing the content of the tadalafil which is an active ingredient to 10 and 20 mg, total weight of solid contents of the film changes but the weight of the surfactant, the plasticizer and the sweetening agent based on total weight of solid contents of the film can be changed while maintaining the above wt%.

The composition for preparing an oral dispersible film of the present disclosure can further selectively include various additives, binding agents, pH controllers, fragrance ingredients, coloring agents, oils, wetting agents, disintegrants and lubricants among others which are generally usable for preparing the oral dispersible film.

The additives are pharmaceutically acceptable additives and can include buffer and diluents among others, and can include a film forming agent for preparing the oral dispersible film. Various macromolecules such as pullulan, gelatin, pectin, low viscosity pectin, hydroxypropyl methylcellulose, low viscosity hydroxypropylmethylcellulose, hydroxyethylcellulose, hydroxypropylcellulose, carboxymethylcellulose, polyvinyl pyrrolidone, polyvinyl alcohol, polyacrylic acid, methyl methacrylate copolymers, carboxyvinyl polymer, polyethylene glycol, alginic acid, low viscosity alginic acid, sodium alginate, starch, casein, whey protein isolate, soy protein isolate, zein, levan, elsinan, gluten, acacia gum, carrageenan, arabic gum, guar gum, locust bean gum, xanthan gum, gellan gum and agar among others are usable as the film forming agent without restriction but the pullulan is most preferable.

The pH controller can be potassium dihydrogen phosphate, sodium hydroxide, sodium hydrogen carbonate, sodium phosphate, ammonium hydroxide, sodium stannate, triethanolamine, citric acid, hydrochloric acid, sodium citrate and combinations thereof and preferably the potassium dihydrogen phosphate and/or the sodium hydroxide can be used. Sufficient pH controller may be added and adjust pH of the film to a proper value.

The fragrance ingredient can be natural fragrance ingredient, synthetic fragrance ingredient or its mixtures thereof without restrictions and preferably extract of leaves, flowers, and fruits of the plant and plant oil. Also, the synthetic fragrance ingredient can be synthetic fruit flavors such as lemon, orange, grape, lime and strawberry, and synthetic flavors such as vanilla, coffee, cocoa, pine needle, ginseng, red ginseng and citrus.

The coloring agent can be titanium oxide, yellow iron oxide, red iron oxide, riboflavin, betacarotene, anthocyan, carmine, indigo carmine, orange yellow s, quinoline yellow, indigotin lake, brilliant blue and sunset yellow.

The oil can be safflower oil, castor oil, coconut oil, cotton seed oil, canola oil, herring oil, palm tree fruit oil, palm oil, peanut oil, soybean oil, rapeseed oil, flaxseed oil, rice bran oil, pine tree oil, sesame oil, sunflower oil, hydrogenated safflower oil and its mixtures thereof.

The disintegrant can be sodium croscarmellose, starch, modified starch, methyl cellulose, carboxymethylcellulose calcium, sodium carboxymethylcellulose, hydroxypropyl cellulose, microcrystalline cellulose, colloidal silicon dioxide, gelatinized starch, mud, cellulose, cellulose powder, sodium alginic acid, alginic acid, guar gum, magnesium aluminum silicate, polacrilin potassium or its mixtures thereof.

The lubricant can be talc, stearic acid, magnesium stearate, colloidal silicon dioxide, and sodium stearyl fumarate, glyceryl behenate, and glyceryl distearate.

In addition, the present disclosure provides the oral dispersible film which is produced by drying the compositions for preparing the oral dispersible film comprising the tadalafil or its pharmaceutically acceptable salt thereof, surfactant, plasticizer and sweetening agent, wherein the surfactant is sodium lauryl sulfate (SLS) and polysorbate, the plasticizer is glycerin and polyethylene glycol and the sweetening agent is sorbitol solution.

Furthermore, the present disclosure provides a method of preparing the oral dispersible film comprising (a) preparing an agitation solution by adding and agitating the tadalafil or its pharmaceutically acceptable salt thereof, the surfactant, the binding agent, the plasticizer, the disintegrant and the sweetening agent into a solvent; (b) preparing the film solution by homogenizing or comminuting the agitation solution with the homomixer, and agitating the same with addition of film forming agent; (c) molding the film by drying the film solution, wherein the surfactant is sodium lauryl sulfate (SLS) and polysorbate, the plasticizer is glycerin and polyethylene glycol and the sweetening agent is sorbitol solution.

The solvent of the step (a) can be one or more solvents selected from a group consisted of purified water,

alcohol, alkyl acetates, dimethyl formamide, dimethyl sulfoxide, acetone, anisole, acetic acid, butyl methyl ether, ethyl ether, ethyl formate, formic acid, pentane, heptane, methyl ethyl keton and methyl isobutylketone.

The film forming agent of the step (b) can be various macromolecules such as pullulan, gelatin, pectin, low viscosity pectin, hydroxypropyl methylcellulose, low viscosity hydroxypropylmethylcellulose, hydroxyethylcellulose, hydroxypropylcellulose, carboxymethylcellulose, polyvinyl pyrrolidone, polyvinyl alcohol, polyacrylic acid, methyl methacrylate copolymers, carboxyvinyl polymer, polyethylene glycol, alginic acid, low viscosity alginic acid, sodium alginate, starch, casein, whey protein isolate, soy protein isolate, zein, levan, elsinan, gluten, acacia gum, carrageenan, arabic gum, guar gum, locust bean gum, xanthan gum, gellan gum and agar among others without restriction but the pullulan is most preferable.

Apropos of the method of preparing the oral dispersible film, dissolution rate of the tadalafil drops when viscosity of the film solution excessively increases and the film is not smoothly formed when viscosity is excessively low, thus difficulties lie in preparing the film.

Additionally, as per the method of preparing the oral dispersible film, dissolution rate of the tadalafil could drop if not using homogenizer, therefore it might be preferable to use the homogenizer for homogenizing the film solution.

In addition, the present disclosure provides the oral dispersible film produced by the method of preparing the oral dispersible film.

The oral dispersible film produced by the method of preparing the oral dispersible film has superiority in markedly increased dissolution rate in oral cavity of tadalafil and inhibition of generation of the related compounds without packaging at the same time.

The present disclosure provides an oral dispersible film produced by drying the compositions for preparing the oral dispersible film as described herein for use in treating sexual dysfunction.

The sexual dysfunction is preferably impotence. The oral dispersible film can be administered to an object and effective amount for preventing or treating the sexual dysfunction can be administered when administering the oral dispersible film to the object.

The numerical values set forth in this embodiment should be construed as comprising range of equivalents unless otherwise specified.

The present disclosure will be described more fully hereinafter with reference to the accompanying examples and comparative examples. However, the present disclosure may be embodied in many different forms, and should not be construed as being limited to the embodiments set forth herein.

[Examples]

**1. Preparation of Example 1**

Following method was conducted to produce the oral dispersible film comprising the tadalafil as an active ingredient. A stirrer (i.e. IKA® EuroStar Digital) was used for production and agitated at speed range of 300rpm through 1,000rpm.

2g of the sodium lauryl sulfate (i.e. SLS, the surfactant) was added to 200g of purified water in a preparation container 1 and completely dissolved by agitation. 20g of the tadalafil was added to the completely dissolved solution and completely dispersed by agitating again. 7g of the polysorbate 20, 7g of the polyethylene glycol 400, 7g of a concentrated glycerin (PALMERA G995) and 7g of the sorbitol solution were added to the prepared half-finished product and agitated again. 6g of the croscarmellose sodium, 12g of the microcrystalline cellulose, 4g of the sucralose and 2g of the titanium oxide were added and agitated again.

Put 2g of L-menthol (i.e. the fragrance ingredient) into a beaker (i.e. preparation container 2) and mixed with 20g of purified water which was warmed over 50°C to be dissolved and added to the preparation container 1.

24g of the pullulan was added and agitated until completely dissolved until being a homogeneous solution and used as the film solution after resting it overnight.

The film containing the tadalafil was obtained through pouring the prepared film solution on a PET film, casting the film solution with a film applicator (YASUDA SEIKI SEISAKUSHO. LTD.), drying the film solution under temperature of over 50°C and exfoliating the film solution from the PET film.

Ingredients used for preparing the film of the present disclosure, and its contents and ratio are shown in Table 1 below. The ratio was calculated as weight of the each ingredient based on total weight of solid contents wherein the purified water as a solvent was excluded, and in case of a formulation containing 10mg of the tadalafil, content was decided by maintaining the ratio of weight of the each ingredient based on total weight of the solid contents.

[Table 1]

| Ingredient | 20mg ingredient/sheet | unit | 10mg ingredient/sheet | unit |
|---|---|---|---|---|
| Tadalafil (principal component) | 20.00 | mg | 10.00 | mg |
| Microcrystalline Cellulose (AvicelPH102) | 12.00 | mg | 6.00 | mg |
| Sucralose | 4.00 | mg | 2.00 | mg |
| Polyethylene Glycol 400 | 7.00 | mg | 3.50 | mg |
| Concentrated Glycerin (PALMERA G995) | 7.00 | mg | 3.50 | mg |
| Sorbitol Solution | 7.00 | mg | 3.50 | mg |
| Polysorbate 20 | 7.00 | mg | 3.50 | mg |
| Sodium Lauryl Sulfate (SLS) | 2.00 | mg | 1.00 | mg |
| Pullulan | 24.00 | mg | 12.00 | mg |
| Sodium Croscarmellose | 6.00 | mg | 3.00 | mg |
| L-Menthol | 2.00 | mg | 1.00 | mg |
| Titanium Oxide | 2.00 | mg | 1.00 | mg |
| Purified water | 220.00 | mg | 110.00 | mg |
| **Total Weight of the Solid Content** | **100.00** | **mg** | **50.00** | **mg** |

[Comparative Examples]

**Preparation of Comparative Examples 1 through 4**

All the other constitutions were identical to those of Example 1 except that the glycerin was excluded in Comparative Example 1, the sorbitol solution was excluded in Comparative Example 2, the polysorbate 20 (Tween20) was excluded in Comparative Example 3 and the sodium lauryl sulfate was excluded in Comparative Example 4, and the oral dispersive film was produced by the same process set forth in Example 1.

Ingredient ratio of each remaining ingredient except the pullulan based on total weight of the solid content was maintained identically to the 20mg formulation of the tadalafil in Example 1.

The oral dispersible film produced with the identical compositions and the process was used in following Experimental Examples.

[Experimental Examples]

**1. Dissolution Rate Analysis**

Dissolution rates of the produced oral dispersible film of Example 1 and Comparative Example 1 through 4 were measured and compared to each other. A method and conditions for measuring the dissolution rate were set as followings according to the test method which is approved by the Korean Ministry of Food and Drug Safety.

- Preparation of a Test Liquid: One sheet of the film was tested with 1000mL of 0.5%sodium dodecyl sulfate (SDS) as a test solution by using a sinker at 50 rpm under temperature range of 37.0 ± 0.5°C according to the second method of dissolution test set forth in the Korean Pharmacopeia. 10ml of eluent was obtained at 10, 30 and 60 minutes after initiating the dissolution test and used as the test liquid by filtering the obtained eluent with 0.45μm membrane filter.

- Preparation of a Standard Solution: 20mg of a reference standard tadalafil was precisely measured by a 100mL volumetric flask, dissolved with a diluents (water-acetonitrile mixture (50 : 50)), filled to the gauge mark and mixed. 5ml of the solution was precisely obtained and poured into the volumetric flask with addition of a test solution by filling the volumetric flask to the mark and mixing the same, and used as the standard solution (0.02mg/mL).

- Operation: Tested with 50μL of the test liquid and the standard solution according to the liquid chromatographic

method of the general tests set forth in the Korean Pharmacopeia and calculated peak area As and At of the tadalafil of the standard solution and the test liquid.

<Operational Condition>

Detector: Ultraviolet Spectrometry (wavelength range: 225nm) Column: ZORBAX SB-C8 (4.6mm x 5cm, 3.5um) or equivalent column Mobile Phase: Methanol-Water Mixture (50 : 50)
Flux: 2.0mL/min
Column Temperature: 40°C

- System Suitability

  - A peak symmetry factor of the tadalafil was 1.5 or less when testing with 50μL of the standard solution under the condition set forth hereinbefore.
  - A relative standard deviation for peak area of the tadalafil was 2.0% or less when conducting six times of a cyclic test with 50μL of the standard solution under the condition set forth hereinbefore.

- Formula

- Ten-minute dissolution rate for indicated amount of the tadalafil in one sheet of the film (%) =

$$\frac{At}{As} \times \frac{Ws}{20} \times P$$

At: Peak Area of the tadalafil in the test liquid
As: Peak area of the tadalafil in the standard solution
Ws: Obtained amount of the reference standard tadalafil (mg)
20: Indicated amount of the tadalafil in one sheet of the film (mg)
P: Purity of the reference standard tadalafil (%)

- Thirty-minute dissolution rate for indicated amount of the tadalafil in one sheet of the film (%) =

$$(Q_{10} \times \frac{v}{V}) + [\frac{At}{As} \times \frac{Ws \times 0.001}{20} \times (V - v) \times P]$$

$Q_{10}$: Ten-minute dissolution rate for indicated amount of the tadalafil in one sheet of the film (%)
v: Volume of the eluent obtained from the ten-minute dissolution test (mL)
V: Volume of the eluent, 1000mL
At: Peak Area of the tadalafil in the test liquid
As: Peak area of the tadalafil in the standard solution
Ws: Obtained amount of the reference standard tadalafil (mg)
0.001: dilution rate of the standard solution (5/(100×50))
20: Indicated amount of the tadalafil in one sheet of the film (mg)
P: Purity of the reference standard tadalafil (%)

- Sixty-minute dissolution rate for indicated amount of the tadalafil in one sheet of the film (%) =

$$(Q_{10} \times \frac{v_1}{V}) + (Q_{30} \times \frac{v_2}{V - v_1}) + [\frac{At}{As} \times \frac{Ws \times 0.001}{20} \times (V - (v_1 + v_2)) \times P]$$

$Q_{10}$: Ten-minute dissolution rate for indicated amount of the tadalafil in one sheet of the film (%)
$Q_{30}$: Thirty-minute dissolution rate for indicated amount of the tadalafil in one sheet of the film (%)
$v_1$: Volume of the eluent obtained from the ten-minute dissolution test (mL)
$v_2$: Volume of the eluent obtained from the thirty-minute dissolution test (mL)
V: Volume of the eluent, 1000mL
At: Peak Area of the tadalafil in the test liquid
As: Peak area of the tadalafil in the standard solution
Ws: Obtained amount of the reference standard tadalafil (mg)
0.001: Dilution rate of the standard solution (5/(100×50))
20: Indicated amount of the tadalafil in one sheet of the film (mg)
P: Purity of the reference standard tadalafil (%)

Result of the dissolution test is shown in Table 2 below

[Table 2]

| Ingredient | | Control Group | Example 1 | Comparative Example 1 | Comparative Example 2 | Comparative Example 3 | Comparative Example 4 |
|---|---|---|---|---|---|---|---|
| Tadalafil | | | 20.00 | 20.00 | 20.00 | 20.00 | 20.00 |
| Microcrystalline cellulose (Avicel PH102) | | Cialis Tablet (Serial No.C043 597) | 12.00 | 12.00 | 12.00 | 12.00 | 12.00 |
| Sucralose | | | 4.00 | 4.00 | 4.00 | 4.00 | 4.00 |
| Polyethylene Glycol 400 | | | 7.00 | 7.00 | 7.00 | 7.00 | 7.00 |
| Concentrated Glycerin (PALMERA G995) | | | 7.00 | 0.00 | 7.00 | 7.00 | 7.00 |
| Sorbitol Solution | | | 7.00 | 7.00 | 0.00 | 7.00 | 7.00 |
| Polysorbate 20 | | | 7.00 | 7.00 | 7.00 | 0.00 | 7.00 |
| Sodium Lauryl Sulfate (SLS) | | | 2.00 | 2.00 | 2.00 | 2.00 | 0.00 |
| Pullulan | | | 24.00 | 31.00 | 31.00 | 31.00 | 26.00 |
| Sodium Croscarmellose | | | 6.00 | 6.00 | 6.00 | 6.00 | 6.00 |
| L-Menthol | | | 2.00 | 2.00 | 2.00 | 2.00 | 2.00 |
| Titanium Oxide | | | 2.00 | 2.00 | 2.00 | 2.00 | 2.00 |
| Total Weight (mg) | | | 100.00 | 100.00 | 100.00 | 100.00 | 100.00 |
| Dissolution Rate (%) | 10 min . | 70.5 | 71.8 | 60.0 | 35.2 | 56.4 | 51.4 |
| | 30 min. | 95.8 | 96.3 | 78.0 | 56.8 | 74.7 | 60.4 |
| | 60 min. | 99.8 | 100.1 | 83.0 | 63.2 | 80.8 | 71.5 |

As shown in Table 2, the cialis tablet and the tadalafil oral dispersible film of the present disclosure (Example 1) exhibited remarkable dissolution rate i.e. 70% or more at 10 minute, 95% or more at 30 minute and 100% at 60 minute, but in Comparative Example 1 through 4 wherein ingredients such as the sodium lauryl sulfate (SLS), the polysorbate 20 (T20), the glycerin, the sorbitol solution among others are excluded, dissolution rate was drastically decreased i.e. 60% or less at 10 minute, 80% or less at 30 minute and 85% or less at 60 minute. In other words, it was understood that a most desirable dissolution pattern was shown when the glycerin, the sorbitol, the polysorbate 20 and the sodium lauryl sulfate (SLS) were mixed respectively or properly.

**2. Related Compounds Analysis**

A related compounds analysis test was conducted to confirm stability of the oral dispersible film in Example 1. A method and conditions for analyzing the related compounds were set as followings according to the test method which is approved by the Korean Ministry of Food and Drug Safety.

- Preparation of a Test Liquid: Put twenty sheets of the film into a 200mL volumetric flask, disintegrate the films by agitating with 80mL of water for fifteen minutes, added 80 mL of acetonitrile and sonicated for five minutes, filled the volumetric flask to the mark with addition of the diluents (acetonitrile-water mixture (50 : 50)) and mixed. 10ml of the solution was precisely obtained and poured into a 100mL volumetric flask, filled the volumetric flask to the mark with addition of the diluents and used as the test liquid by filtering the mixed solution via 0.45μm membrane filter.

- Preparation of a Standard Solution: 20mg of the reference standard tadalafil was precisely measured and put into the 100mL volumetric flask, dissolved by diluents and used as the standard solution (0.2mg/mL) by filling the volumetric flask to the mark and mixing.

- Preparation of a System Suitability Solution: To transform the tadalafil to 6R and 12aS (i.e. diastereomeric), obtained 25mL of the standard solution and poured into a 100mL beaker, added 0.25mL of 5mol/L sodium hydroxide solution and agitated, and rested for thirty minutes. This solution was used as the system suitability solution via neutralization to pH 7.0 by using trifluoroacetic acid. Relative retention time was 1.0 for the tadalafil and 1.2 for the 6R, 12aS.

- Detection Solution: 50uL of the standard solution was precisely obtained and poured into the 100mL volumetric flask and used as the detection solution (0.1μg/mL) by filling the volumetric flask to the mark with diluents.

- Operation: Tested with 10μL of the test liquid, the standard solution and the system suitability solution according to the liquid chromatographic method of the general tests set forth in the Korean Pharmacopeia and calculated peak area As and At of the tadalafil of the standard solution and the test liquid.

<Operational Condition>

Detector: Ultraviolet Spectrometry (wavelength range: 285nm)
Column: ZORBAX SB-C8 (4.6mm x 5cm, 3.5um) or equivalent column
Mobile Phase: Acetonitrile-Water-Trifluoroacetic acid Mixture (35 : 65 : 0.1)
Flux: 1.0mL/min
Column Temperature: 35°C

- System Suitability
- Resolution of the tadalafil and the 6R,12aS (i.e. diastereomeric) was greater than 3.0 when testing with 10μL of the system suitability solution under the condition set forth hereinbefore.
- A relative standard deviation for peak area of the tadalafil was 2.0% or less and symmetry factor was 2.0 or less when conducting six times of a cyclic test with 10μL of the standard solution under the condition set forth hereinbefore.
- A ratio of signal to noise (S/N) of the detection solution was greater than 10.
- Formula

$$\text{Individual related compound (} \frac{At}{As} \times 100$$

At: Peak Area of the individual related compound in the test liquid
As: Aggregate Peak Areas of the individual related compound and the tadalafil in the test liquid
*Values under 0.05% (limit of quantification, equivalent to 0.1μg/mL) were excluded.

At the related compounds analysis, stability was deemed to have no problems when each individual related compound was 0.2% or less and total related compounds were 0.3% or less. To analyze the related compounds more specifically, stored the packed or unpacked oral dispersible films of Example 1 in an oven at temperature of 80°C and followed the related compounds analysis set forth hereinbefore. Cialis tablet which is a tadalafil tablet on sale was used as a control group and tested for packed or unpacked status under identical conditions. The related compounds analysis has been conducted 3 times in 15 days and the analysis result is shown in Tables 3 and 4.

[Table 3]

| Generation of the related compounds under packed status | | | | | | |
|---|---|---|---|---|---|---|
| | | Initial | Day 1 | Day 3 | Day 9 | Day 15 |
| Example 1 | Tadalafil (%) | 99.85 | 99.95 | 99.94 | 99.89 | 99.84 |
| | Total Related Compounds (%) | 0.01 | 0.01 | 0.03 | 0.06 | 0.11 |
| Cialis | Tadalafil (%) | 99.92 | 99.76 | 99.56 | 99.39 | 99.33 |
| | Total Related Compounds (%) | 0.07 | 0.20 | 0.38 | 0.56 | 0.6 |

[Table 4]

| Generation of the related compounds under unpacked status | | | | | | | |
|---|---|---|---|---|---|---|---|
| | | Initial | Day 1 | Day 3 | Day 7 | Day 11 | Day 15 |
| Example 1 | Tadalafil (%) | 99.94 | 99.96 | 99.95 | 99.92 | 99.89 | 99.77 |
| | Total Related Compounds (%) | 0.00 | 0.01 | 0.01 | 0.03 | 0.05 | 0.12 |
| Cialis | Tadalafil (%) | 99.92 | 99.90 | 99.87 | 99.81 | 99.74 | 99.68 |
| | Total Related Compounds (%) | 0.04 | 0.07 | 0.10 | 0.15 | 0.20 | 0.23 |

As shown in Tables 3 and 4, it was confirmed that the tadalafil oral dispersible film of Example 1 was a formulation having superior stability by the result showing that the total related compounds were generated less than the threshold of 0.3% even after 15 days under both packed and unpacked status. In contrast, the cialis tablet i.e., the control group, did not meet the stability criteria because the total related compounds increased up to 0.6% after 15 days under packed status. Therefore, the tadalafil oral dispersible film of Example 1 was confirmed as an effective formulation which could be a solution to the previous cialis tablets having stability problem.

### 3. Property Retention Analysis

It is crucial to retain properties of the oral dispersible film which is administered by dissolving in oral cavity during distribution after production. Especially, its wide surface area and thin thickness compared to the tablets or capsules can incur breakage or problematic flexibility. Also, due to use of lots of liquid additives, thickness of the film becomes thinner when water among others vapors and leakage of the liquid additives to the surface incurs. To resolve these problems, retention of the properties was observed through variation of amount of the polyethylene glycol 400 (i.e. PEG 400, the plasticizer).

More specifically, prepared the tadalafil oral dispersible film while varying only the content of the polyethylene glycol 400 to 1% through 9% based on total weight and observed differences of oil leakage, breakage and flexibility of the film. The prepared films are shown as Examples 1 through 5 and Comparative Example 5. 64 days of storage status was observed in a storage chamber fixed at 80°C.

[Table 5]

| Pre and Post-properties of the film storage in the 80°C chamber. | | | | | | | |
|---|---|---|---|---|---|---|---|
| **Ingredient** | | **Example 2** | **Example 3** | **Example 4** | **Example 1** | **Example 5** | **Comparative Example 5** |
| Tadalafil | | 20.00 | 20.00 | 20.00 | 20.00 | 20.00 | 20.00 |
| Microcrystalline Cellulose (Avicel PH102) | | 12.00 | 12.00 | 12.00 | 12.00 | 0.00 | 12.00 |
| Sucralose | | 4.00 | 4.00 | 4.00 | 4.00 | 4.00 | 4.00 |
| **Polyethylene Glycol 400** | | **1.00** | **3.00** | **5.00** | **7.00** | **7.00** | **9.00** |
| Concentrated Glycerin (PALMERA G995) | | 7.00 | 7.00 | 7.00 | 7.00 | 7.00 | 7.00 |
| Sorbitol Solution | | 7.00 | 7.00 | 7.00 | 7.00 | 7.00 | 7.00 |
| Polysorbate 20 | | 7.00 | 7.00 | 7.00 | 7.00 | 7.00 | 7.00 |
| Sodium Lauryl Sulfate (SLS) | | 2.00 | 2.00 | 2.00 | 2.00 | 2.00 | 2.00 |
| Pullulan | | 30.00 | 28.00 | 26.00 | 24.00 | 36.00 | 22.00 |
| Sodium Croscarmellose | | 6.00 | 6.00 | 6.00 | 6.00 | 6.00 | 6.00 |
| L-Menthol | | 2.00 | 2.00 | 2.00 | 2.00 | 2.00 | 2.00 |
| Titanium Oxide | | 2.00 | 2.00 | 2.00 | 2.00 | 2.00 | 2.00 |
| Total Weight | | 100.00 | 100.00 | 100.00 | 100.00 | 100.00 | 100.00 |
| Storage (days) | | Oil Observation on Storage Status of the Film | | | | | |
| Initial | Observed | Not observed | Not observed | Not observed | Not observed | Not observed | Not observed |
| 1 | Observed | Not observed | Not observed | Not observed | Not observed | Not observed | Observed |
| 4 | Observed | Not observed | Not observed | Not observed | Not observed | Not observed | Observed |
| 8 | Observed | Not observed | Not observed | Not observed | Not observed | Not observed | Observed |
| 15 | Observed | Not observed | Not observed | Not observed | Not observed | Not observed | Observed |
| 36 | Observed | Not observed | Not observed | Not observed | Not observed | Not observed | Dried |
| 64 | Observed | Not observed | Not observed | Not observed | Not observed | Not observed | Dried |

According to the result shown in Table 5, no oil leakage from the film formulation was observed when usage of the polyethylene glycol 400 was in range of 1.0% through 7.0% based on total weight. However, when the content of the polyethylene glycol 400 was 9% (i.e. Comparative Example 5), oil leakage was observed from the initial day and dried from $36^{th}$ day of observation and accordingly confirmed influences on the products caused by stains remaining on the film surface.

Additional observation on whether the above prepared tadalafil oral dispersible film has superior properties in oil leakage, flexibility and breakage was conducted and shown in Table 6 below.

[Table 6]

| Storage (days) | Breakage(B) and Flexibility(F) of the Film | | | | | | | | | |
|---|---|---|---|---|---|---|---|---|---|---|
| | Example 2 | | Example 3 | | Example 4 | | Example 1 | | Example 5 | |
| | B | F | B | F | B | F | B | F | B | F |
| Initial | X | ○ | X | ○ | X | ○ | X | ○ | X | ○ |
| 1 | X | ○ | X | ○ | X | ○ | X | ○ | X | ○ |
| 4 | X | ○ | X | ○ | X | ○ | X | ○ | X | ○ |
| 8 | X | ○ | X | ○ | X | ○ | X | ○ | X | ○ |
| 15 | X | ○ | X | ○ | X | ○ | X | ○ | X | ○ |
| 36 | Δ | Δ | X | ○ | X | ○ | X | ○ | X | ○ |
| 64 | Δ | Δ | Δ | Δ | Δ | Δ | Δ | Δ | Δ | Δ |
| X: No Breakage, Δ: sporadic Breakage, 0: Breakage | | | | | | | | | | |
| X: Low Flexibility, Δ: Normal Flexibility, 0: High Flexibility | | | | | | | | | | |

As shown in Table 6, the tadalafil film formulations of the present disclosure all barely exhibited breakage and had satisfactory flexibility under the circumstance of long-term storage. Therefore, it was confirmed that the tadalafil oral dispersible film comprising 1-7 wt% of the polyethylene glycol 400 based on total weight of the film could maintain superior film properties in long-term comprising no breakage, satisfactory flexibility and no oil leakage.

## Claims

1. A composition for preparing an oral dispersible film comprising tadalafil or a pharmaceutically acceptable salt thereof, a surfactant, a plasticizer and a sweetening agent wherein the surfactant is sodium lauryl sulfate (SLS) and polysorbate, the plasticizer is glycerin and polyethylene glycol and the sweetening agent is sorbitol solution.

2. The composition for preparing the oral dispersible film according to claim 1 wherein the surfactant is comprised 1-10 wt% based on total solid content of the film.

3. The composition for preparing the oral dispersible film according to claim 1, wherein the plasticizer is the glycerin and a polyethylene glycol 400.

4. The composition for preparing the oral dispersible film according to claim 3, wherein the polyethylene glycol 400 is comprised 1-7 wt% based on total solid content of the film.

5. The composition for preparing the oral dispersible film according to claim 1, wherein the sweetening agent is comprised 1-30 wt% based on total solid content of the film.

6. The oral dispersible film produced by drying the composition according to any one of the claims 1-5.

7. A method of preparing the oral dispersible film comprising (a) preparing an agitation solution by adding and agitating the tadalafil or its pharmaceutically acceptable salt thereof, the surfactant, a binding agent, the plasticizer, a disintegrant and the sweetening agent into a solvent; (b) preparing a film solution by homogenizing or comminuting the agitation solution with a homomixer, and agitating the same with addition of a film forming agent; and (c) molding the film by drying the film solution wherein the surfactant is sodium lauryl sulfate (SLS) and polysorbate, the plasticizer is glycerin and polyethylene glycol and the sweetening agent is sorbitol solution.

8. The method of preparing the oral dispersible film according to claim 7, wherein the polyethylene glycol is comprised as 1-7 wt% based on total weight of solid contents of the film.

## Patentansprüche

1. Zusammensetzung zur Herstellung eines oral dispergierbaren Films, der Tadalafil oder ein pharmazeutisch annehmbares Salz davon, ein Tensid, ein Plastifizierungsmittel und ein Süßungsmittel umfasst, wobei das Tensid Natriumlaurylsulfat (SLS) und Polysorbat ist, das Plastifizierungsmittel Glycerin und Polyethylenglykol ist, und das Süßungsmittel Sorbitlösung ist.

2. Zusammensetzung zur Herstellung des oral dispergierbaren Films nach Anspruch 1, wobei das Tensid 1 bis 10 Gew.% stellt, bezogen auf den Gesamtfeststoffgehalt des Films.

3. Zusammensetzung zur Herstellung des oral dispergierbaren Films nach Anspruch 1, wobei das Plastifizierungsmittel das Glycerin und ein Polyethylenglykol 400 ist.

4. Zusammensetzung zur Herstellung des oral dispergierbaren Films nach Anspruch 3, wobei das Polyethylenglykol 400 1 bis 7 Gew.% stellt, bezogen auf den Gesamtfeststoffgehalt des Films.

5. Zusammensetzung zur Herstellung des oral dispergierbaren Films nach Anspruch 1, wobei das Süßungsmittel 1 bis 30 Gew.% stellt, bezogen auf den Gesamtfeststoffgehalt des Films.

6. Oral dispergierbarer Film, der durch Trocknen der Zusammensetzung gemäß einem der Ansprüche 1 bis 5 produziert wird.

7. Verfahren zur Herstellung des oral dispergierbaren Films, umfassend (a) Herstellen einer Rührlösung durch Zufügen und Rühren von Tadalafil oder dessen pharmazeutisch annehmbarem Salz, dem Tensid, einem Bindungsmittel, dem Plastifizierungsmittel, einem Sprengmittel und dem Süßungsmittel in einem Lösungsmittel; (b) Herstellen einer Filmlösung durch Homogenisieren oder Zerkleinern der Rührlösung mit einem Homomixer und Rühren derselben unter Zugabe eines Filmbildungsmittels; und (c) Formen des Films durch Trocknen der Filmlösung, wobei das Tensid Natriumlaurylsulfat (SLS) und Polysorbat ist, das Plastifizierungsmittel Glycerin und Polyethylenglykol ist, und das Süßungsmittel Sorbitlösung ist.

8. Verfahren zur Herstellung des oral dispergierbaren Films nach Anspruch 7, wobei das Polyethylenglykol 1 bis 7 Gew.% stellt, bezogen auf den Gesamtfeststoffgehalt des Films.

## Revendications

1. Composition pour la préparation d'un film dispersible oral comprenant du tadalafil ou un sel pharmaceutiquement acceptable correspondant, un tensioactif, un plastifiant et un agent sucrant, le tensioactif étant le laurylsulfate de sodium (SLS) et un polysorbate, le plastifiant étant la glycérine et un polyéthylène glycol et l'agent sucrant étant une solution de sorbitol.

2. Composition pour la préparation d'un film dispersible oral selon la revendication 1, le tensioactif représentant 1 à 10 % en poids sur la base de la teneur totale en solides du film.

3. Composition pour la préparation d'un film dispersible oral selon la revendication 1, le plastifiant étant la glycérine et un polyéthylène glycol 400.

4. Composition pour la préparation d'un film dispersible oral selon la revendication 3, le polyéthylène glycol 400 représentant 1 à 7 % en poids sur la base de la teneur totale en solides du film.

5. Composition pour la préparation d'un film dispersible oral selon la revendication 1, l'agent sucrant représentant 1 à 30 % en poids sur la base de la teneur totale en solides du film.

6. Film dispersible oral produit par séchage de la composition selon l'une quelconque des revendications 1 à 5.

7. Procédé de préparation du film dispersible oral comprenant (a) la préparation d'une solution d'agitation par ajout et agitation du tadalafil ou de son sel pharmaceutiquement acceptable correspondant, du tensioactif, d'un agent liant, du plastifiant, d'un désintégrant et de l'agent sucrant dans un solvant ; (b) la préparation d'une solution de film par

homogénéisation ou par broyage de la solution d'agitation avec un homomixeur, et agitation de celle-ci avec ajout d'un agent de formation de film ; et (c) le moulage du film par séchage de la solution de film, le tensioactif étant le laurylsulfate de sodium (SLS) et un polysorbate, le plastifiant étant la glycérine et un polyéthylène glycol et l'agent sucrant étant une solution de sorbitol.

**8.** Procédé de préparation du film dispersible oral selon la revendication 7, le polyéthylène glycol représentant 1 à 7 % en poids sur la base du poids total des teneurs en solides du film.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6841167 B
- KR 20080023873
- WO 2012053006 A
- US 2010240724 A
- WO 2014091134 A
- EP 3111929 A


### Non-patent literature cited in the description


- *CHEMICAL ABSTRACTS,* 171596-29-5